# EUROPEAN PATENT APPLICATION

(11) **EP 2 353 714 A1**
(43) Date of publication of application: **10.08.2011**
(21) Application number: 09829154.5
(22) Date of filing: 27.11.2009
(51) Int. Cl.: B01J 31/28, C07C 45/62, C07C 47/02, C07B 53/00, C07B 61/00

(54) **ASYMMETRIC HYDROGENATION CATALYST**

(30) Priority: 27.11.2008 JP 2008301829
(71) Applicant: Takasago International Corporation, Tokyo-to 144-8721 (JP)
(72) Inventor: MAEDA Hironori, Hiratsuka-shi Kanagawa 254-0073 (JP); HORI Yoji, Hiratsuka-shi Kanagawa 254-0073 (JP)
(74) Representative: Zimmermann & Partner
(86) International application number: PCT/JP2009/070005
(87) International publication number: WO 2010/061909

(57) **Abstract**

Provided is a catalyst for producing an optically active aldehyde by selective asymmetric hydrogenation of an α, β-unsaturated aldehyde, particularly a catalyst that is insoluble in the reaction mixture and is used to obtain optically active citronellal, which is useful as a fragrance, by selective asymmetric hydrogenation of citral, geranial or neral. Also provided is a method for producing the corresponding optically active aldehyde. The α, β-unsaturated aldehyde is subjected to asymmetric hydrogenation using a solid catalyst comprising an optically active compound and a metal-supporting product wherein a carrier supports at least one metal selected from the group consisting of the elements in Groups 8 through 10 of the Periodic Table.

## Description

### TECHNICAL FIELD

The present invention relates to a method for producing an optically active aldehyde by selective asymmetric hydrogenation of an carbon-carbon double bond of an α,β-unsaturated aldehyde by using a solid catalyst for asymmetric hydrogenation of an α,β-unsaturated aldehyde. Particularly, selective asymmetric hydrogenation of an α,β-unsaturated carbon-carbon double bond of citral (a mixture of geranial and neral), geranial, or neral can provide optically active citronellal useful as a flavor and/or fragrance.

### BACKGROUND ART

Conventionally, attempts have been made for asymmetric hydrogenation of a carbon-carbon double bond of an α,β-unsaturated aldehyde with hydrogen gas. Particularly, there are known methods for asymmetric hydrogenation of neral or geranial to obtain optically active citronellal, which is important as a flavor and/or a fragrance (Patent Documents 1 and 2). In these methods, the carbon-carbon double bond is hydrogenated with hydrogen gas by using a small amount of a homogeneous catalyst. Accordingly, no auxiliary agent is necessary, and hence a large amount of waste products are not generated.

### PRIOR ART DOCUMENTS

### PATENT DOCUMENTS

Patent Document 1: Japanese Patent Application Publication No. Sho 54-14911.
Patent Document 2: Published Japanese Translation of PCT International Application No. 2008-515843.

### SUMMARY OF THE INVENTION

However, catalysts used in the above-described methods are homogeneous catalysts using expensive rhodium metal or the like. Hence, the catalysts are soluble in a reaction solution and are difficult to recover.

In this respect, it is conceivable that the use of a heterogeneous catalyst such as a solid catalyst insoluble in a reaction solution facilitates the recovery of the catalyst. However, asymmetric hydrogenation reaction of an α,β-unsaturated aldehyde using a solid catalyst has not been known.

An object of the present invention is to provide a method for obtaining a corresponding optically active aldehyde by asymmetric hydrogenation of a carbon-carbon double bond of an α,β-unsaturated aldehyde by using a solid catalyst for asymmetric hydrogenation which can be easily separated from a reaction solution. Particularly, an object of the present invention is to provide a method for obtaining optically active citronellal by hydrogenating citral, geranial, or neral through asymmetric hydrogenation reaction.

The present inventors have made earnest studies to solve the above described problems. As a result, the present inventors have found that when asymmetric hydrogenation of an α,β-unsaturated aldehyde is conducted by using a solid catalyst containing a specific optically active compound and a specific metal-supporting material, a corresponding optically active aldehyde can be obtained. This finding led to the completion of the present invention. In addition, the optically active compound and the metal-supporting material are recovered from the system after completion of the reaction, and then can be reused as the asymmetric hydrogenation catalyst.

Specifically, the present invention includes the following inventions.
[1] A solid catalyst for asymmetric hydrogenation of an α,β-unsaturated aldehyde, including: a metal-supporting material in which at least one metal selected from the group consisting of elements of Groups 8 to 10 of a periodic table is supported on a support; and an optically active compound.
[2] The solid catalyst according to [1], further including an organic base, an organic acid salt, or an inorganic base.
[3] The solid catalyst according to [1] or [2], wherein the optically active compound is an amino acid, an amino alcohol, a salt of an amino acid and an acid, or a salt of an amino alcohol and an acid.
[4] The solid catalyst according to any one of [1] to [3], wherein the metal is selected from the group consisting of nickel, ruthenium, rhodium, palladium, and platinum.
[5] A method for producing an optically active aldehyde, comprising the step of performing asymmetric hydrogenation of an α,β-unsaturated aldehyde by using the solid catalyst according to any one of [1] to [4], the α,β-unsaturated aldehyde being represented by the following general formula (1): 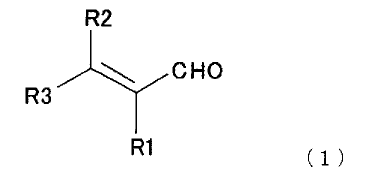 wherein R¹, R² and R³ each independently represent a hydrogen atom, an alkyl group which may have a substituent, a cycloalkyl group which may have a substituent, an alkenyl group which may have a substituent, or an aryl group which may have a substituent; R¹ and R³, or R² and R³ may form a ring, provided that R² and R³ are mutually different groups, and that two groups of R¹, R², and R³ are not simultaneously hydrogen atoms, the optically active aldehyde being represented by the following general formula (2): 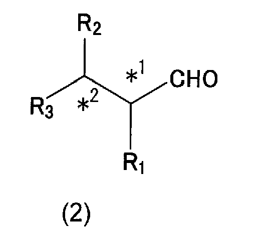 wherein R¹, R² and R³ have the same meanings as those in the formula (1); and *¹ and *² represent asymmetric carbon atoms; provided that when R¹ is hydrogen, *¹ is not an asymmetric carbon atom, and that when R² or R³ is hydrogen, *² is not an asymmetric carbon atom.
[6] The production method according to [5], wherein the α,β-unsaturated aldehyde is geranial, neral, or citral (a mixture of geranial and neral).

As described above, in the present invention, the optically active compound, which serves as an additive contributing to the enantioselectivity, is used as the catalyst for asymmetric hydrogenation reaction, together with the metal-supporting material. Moreover, a catalyst modification step is no more necessary, and the asymmetric hydrogenation is conducted by simply mixing a raw material compound, the optically active compound, and the metal-supporting material. In this manner, the operations are simple, and the metal-supporting material can be recovered and reused, which are industrially advantageous.

### DESCRIPTION OF EMBODIMENTS

Hereinafter, the present invention will be described in detail. In the present invention, an α,β-unsaturated aldehyde is used as a substrate, and is subjected to asymmetric hydrogenation to produce an optically active aldehyde. The α,β-unsaturated aldehyde used as the substrate is not particularly limited, and examples thereof include compounds represented by the following general formula (1). Note that when the double bond at the α- and β-positions of the α,β-unsaturated aldehyde can be in a cis configuration or a trans configuration, both of such α,β-unsaturated aldehydes in the cis configuration and the trans configuration are included in the example. 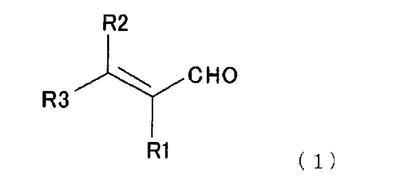 where R¹, R² and R³ each independently represent a hydrogen atom, an alkyl group which may have a substituent, a cycloalkyl group which may have a substituent, an alkenyl group which may have a substituent, or an aryl group which may have a substituent; R¹ and R³, or R² and R³ may form a ring, provided that R² and R³ are mutually different groups, and that two groups of R¹, R², and R³ are not simultaneously hydrogen atoms.

By asymmetric hydrogenation of the compound represented by the formula (1), a compound (optically active aldehyde) represented by the following formula (2) is produced: 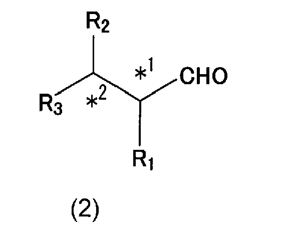 where R¹, R² and R³ represent the same substituents as in the formula (1); and *¹ and *² represent asymmetric carbon atoms; provided that when R¹ is hydrogen, *¹ is not an asymmetric carbon atom, and that when R² or R³ is hydrogen, *² is not an asymmetric carbon atom.

Examples of the group represented by R¹, R² or R³ in the α,β-unsaturated aldehyde represented by the general formula (1) include alkyl groups, cycloalkyl groups, alkenyl groups, and aryl groups. Any of those groups may be substituted with a substituent.

Examples of the alkyl group represented by R¹, R², or R³ include linear or branched alkyl groups having, for example, 1 to 30 carbon atoms, preferably 1 to 10 carbon atoms. Specific examples of the alkyl group include a methyl group, an ethyl group, a n-propyl group, an isopropyl group, a n-butyl group, a 2-butyl group, an isobutyl group, a tert-butyl group, a n-pentyl group, a 2-pentyl group, a 3-pentyl group, a tert-pentyl group, a 2-methylbutyl group, a 3-methylbutyl group, a 2,2-dimethylpropyl group, a 1,2-dimethylpropyl group, a n-hexyl group, a 2-hexyl group, a 3-hexyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, and the like.

These alkyl groups may have substituents, and examples of the substituents of the alkyl groups include alkenyl groups, alkynyl groups, aryl groups, aliphatic heterocyclic groups, aromatic heterocyclic groups, alkoxy groups, alkylenedioxy groups, aryloxy groups, aralkyloxy groups, heteroaryloxy groups, substituted amino groups, halogen atoms, and the like.

The alkenyl group as a substituent of the alkyl group may be linear or branched, and examples thereof include alkenyl groups having 2 to 20 carbon atoms, preferably 2 to 10 carbon atoms, and more preferably 2 to 6 carbon atoms. Specific examples thereof include a vinyl group, a propenyl group, a 1-butenyl group, a pentenyl group, a hexenyl group, and the like.

The alkynyl group as a substituent of the alkyl group may be linear or branched, and examples thereof include alkynyl groups having 2 to 15 carbon atoms, preferably 2 to 10 carbon atoms, and more preferably 2 to 6 carbon atoms. Specific examples thereof include an ethynyl group, a 1-propynyl group, a 2-propynyl group, a 1-butynyl group, a 3-butynyl group, a pentynyl group, a hexynyl group, and the like.

Examples of the aryl group as a substituent of the alkyl group include aryl groups having 6 to 14 carbon atoms, and specific examples thereof include a phenyl group, a naphthyl group, an anthryl group, a phenanthryl group, a biphenyl group, a tolyl group, a xylyl group, a mesityl group, a methoxyphenyl group, a dimethoxyphenyl group, a fluorophenyl group, and the like.

Examples of the aliphatic heterocyclic group as a substituent of the alkyl group include 5- to 8-membered, preferably 5- or 6-membered monocyclic aliphatic heterocyclic groups and polycyclic or fused cyclic aliphatic heterocyclic groups having 2 to 14 carbon atoms and containing, as the hetero atom, at least one, preferably 1 to 3 hetero atoms such as nitrogen atoms, oxygen atoms, and sulfur atoms. Specific examples of the aliphatic heterocyclic group include a 2-oxo-1-pyrrolidinyl group, a piperidino group, a piperazinyl group, a morpholino group, a tetrahydrofuryl group, a tetrahydropyranyl group, a tetrahydrothienyl group, and the like.

Examples of the aromatic heterocyclic group as a substituent of the alkyl group include 5- to 8-membered, preferably 5- or 6-membered monocyclic aromatic heterocyclic groups and polycyclic or fused cyclic aromatic heterocyclic groups having 2 to 15 carbon atoms and containing, as the hetero atom, at least one, preferably 1 to 3 hetero atoms such as nitrogen atoms, oxygen atoms, and sulfur atoms. Specific examples thereof include a furyl group, a thienyl group, a pyridyl group, a pyrimidinyl group, a pyrazinyl group, a pyridazinyl group, a pyrazolinyl group, a imidazolyl group, an oxazolinyl group, a thiazolinyl group, a benzofuryl group, a benzothienyl group, a quinolyl group, an isoquinolyl group, a quinoxalinyl group, a phthalazinyl group, a quinazolinyl group, a naphthyridinyl group, a cinnolinyl group, a benzoimidazolyl group, a benzoxazolyl group, a benzothiazolyl group, and the like.

Examples of the alkoxy group as a substituent of the alkyl group include linear or branched alkoxy groups having 1 to 6 carbon atoms. Specific examples thereof include a methoxy group, an ethoxy group, a n-propoxy group, an isopropoxy group, a n-butoxy group, a 2-butoxy group, an isobutoxy group, a tert-butoxy group, a n-pentyloxy group, a 2-methylbutoxy group, a 3-methylbutoxy group, a 2,2-dimethylpropyloxy group, a n-hexyloxy group, a 2-methylpentyloxy group, a 3-methylpentyloxy group, a 4-methylpentyloxy group, a 5-methylpentyloxy group, and the like.

Examples of the alkylenedioxy group as a substituent of the alkyl group include alkylenedioxy groups having 1 to 3 carbon atoms. Specific examples thereof include a methylenedioxy group, an ethylenedioxy group, a propylenedioxy group, an isopropylidenedioxy group, and the like.

Examples of the aryloxy group as a substituent of the alkyl group include aryloxy groups having 6 to 14 carbon atoms. Specific examples thereof include a phenyloxy group, a naphthyloxy group, an anthryloxy group, and the like.

Examples of the aralkyloxy group as a substituent of the alkyl group include aralkyloxy groups having 7 to 12 carbon atoms. Specific examples thereof include a benzyloxy group, a 2-phenylethoxy group, a 1-phenylpropoxy group, a 2-phenylpropoxy group, a 3-phenylpropoxy group, a 1-phenylbutoxy group, a 2-phenylbutoxy group, a 3-phenylbutoxy group, a 4-phenylbutoxy group, a 1-phenylpentyloxy group, a 2-phenylpentyloxy group, a 3-phenylpentyloxy group, a 4-phenylpentyloxy group, a 5-phenylpentyloxy group, a 1-phenylhexyloxy group, a 2-phenylhexyloxy group, a 3-phenylhexyloxy group, a 4-phenylhexyloxy group, a 5-phenylhexyloxy group, a 6-phenylhexyloxy group, and the like.

Examples of the heteroaryloxy group as a substituent of the alkyl group include heteroaryloxy groups having 2 to 14 carbon atoms and containing, as the hetero atom, at least one, preferably 1 to 3 hetero atoms such as nitrogen atoms, oxygen atoms, and sulfur atoms. Specific examples thereof include a 2-pyridyloxy group, a 2-pyrazyloxy group, a 2-pyrimidyloxy group, a 2-quinolyloxy group, and the like.

Examples of the substituted amino group as a substituent of the alkyl group include mono- or di-alkylamino groups (the number of carbon atoms of each alkyl group is, for example, 1 to 30, and preferably 1 to 10) such as an N-methylamino group, an N,N-dimethylamino group, an N,N-diethylamino group, an N,N-diisopropylamino group, and an N-cyclohexylamino group; mono- or di-arylamino groups (the number of carbon atoms of each aryl group is, for example, 6 to 14) such as an N-phenylamino group, an N,N-diphenylamino group, an N-naphthylamino group, and an N-naphthyl-N-phenylamino group; mono- or di-aralkylamino groups (the number of carbon atoms of each aralkyl group is, for example, 7 to 15) such as an N-benzylamino group and an N,N-dibenzylamino group; and the like.

Examples of the halogen atom as a substituent of the alkyl group include a fluorine atom, a chlorine atom, a bromine atom, an iodine atom, and the like.

Examples of the cycloalkyl group represented by R¹, R² or R³ include cycloalkyl groups having 3 to 10 carbon atoms, preferably 3 to 7 carbon atoms. Specific examples thereof include a cyclopropyl group, a cyclobutyl group, a cyclopentyl group, a cyclohexyl group, and the like. These cycloalkyl groups may have substituents, and examples of the substituents include the aforementioned substituents described as the substituents of the alkyl group; and alkyl groups (the number of carbon atoms of each alkyl group is, for example, 1 to 6).

Examples of the alkenyl group represented by R¹, R² or R³ include may be linear, branched or cyclic alkenyl groups having 2 to 20 carbon atoms, preferably 2 to 10 carbon atoms. Specific examples of the alkenyl group include a vinyl group, a 1-propenyl group, a 2-propenyl group, a 1-butenyl group, a 2-butenyl group, a 3-butenyl group, a 1-pentenyl group, a 2-pentenyl group, a 3-pentenyl group, a 4-pentenyl group, a 1-cyclopentenyl group, a 3-cyclopentenyl group, a 1-hexenyl group, a 2-hexenyl group, a 3-hexenyl group, a 4-hexenyl group, a 5-hexenyl group, a 4-methyl-3-pentenyl group, a 4,8-dimethyl-3,7-nonadienyl group, a 1-cyclohexenyl group, a 3-cyclohexenyl group, and the like.

These alkenyl groups may have substituents, and examples of the substituents include the aforementioned groups described as the substituents of the alkyl group; and alkyl groups (the number of carbon atoms of each alkyl group is, for example, 1 to 6).

Examples of the aryl group represented by R¹, R² or R³ include aryl groups having 6 to 14 carbon atoms. Specific examples thereof include a phenyl group, a naphthyl group, an anthryl group, a phenanthryl group, a biphenyl group, and the like. These aryl groups may have substituents, and examples of the substituents include the aforementioned groups described as the substituents of the alkyl group; and alkyl groups (the number of carbon atoms of each alkyl group is, for example, 1 to 6).

Examples of the ring formed by R¹ and R³, or R² and R³ include alicyclic groups having 3 to 10 carbon atoms, and specific examples thereof include a cyclopentane ring, a cyclohexane ring, a cyclopentene ring, a cyclohexene ring, an octahydronaphthalene ring, a decahydronaphthalene ring, and the like. These rings may be substituted with the above-described alkyl groups, acyl groups, and the like.

Specific examples of the α,β-unsaturated aldehyde used as the substrate in the present invention include the following compounds. Note that when the double bond at the α- and β-positions of the α,β-unsaturated aldehyde can be in a cis configuration or a trans configuration, both of such α,β-unsaturated aldehydes in the cis configuration and the trans configuration are included in the example. In the specific examples, each wavy line represents all of a cis configuration, a trans configuration, and a mixture thereof.

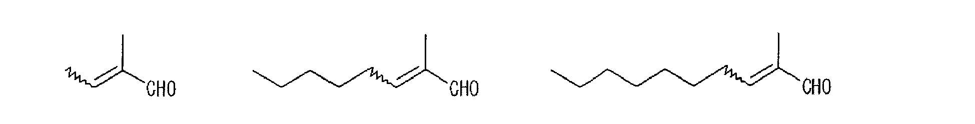

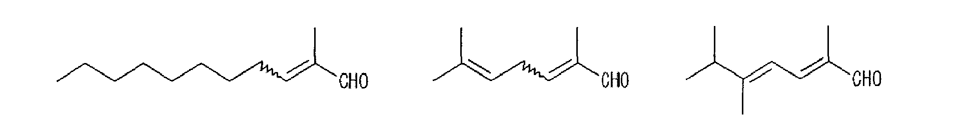

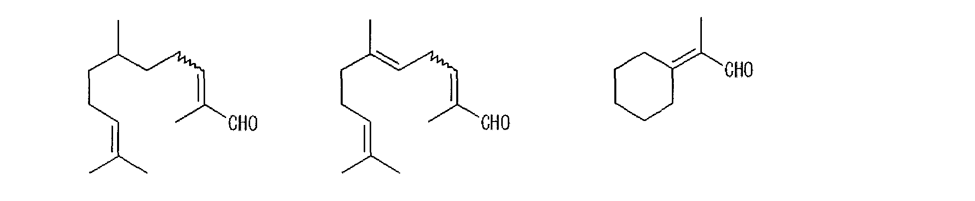

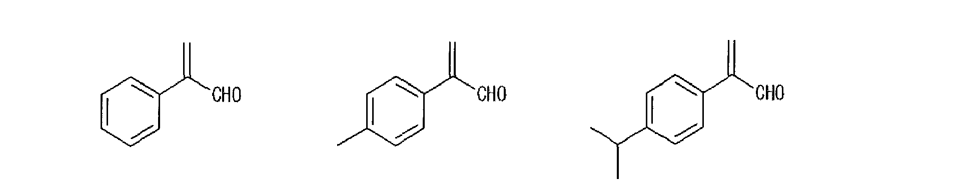

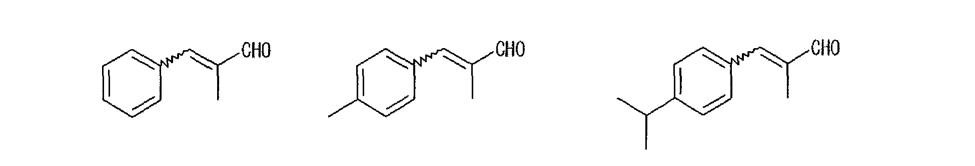

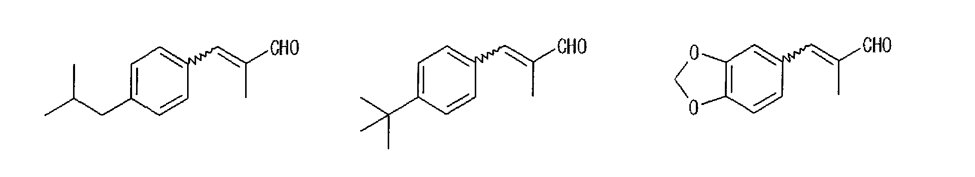

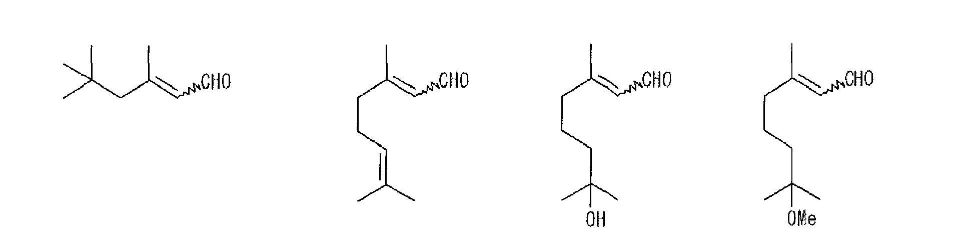

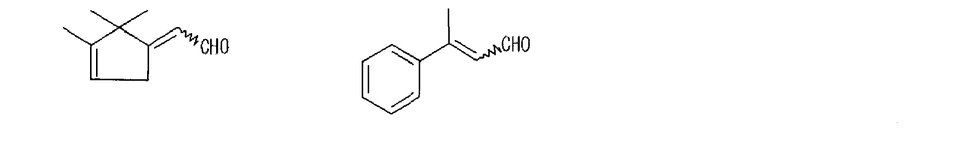

Note that, among the above α,β-unsaturated aldehydes, geranial, neral, and citral are particularly preferable.

Next, description will be given of the metal-supporting material used as a catalyst component in the method of the present invention. Preferred examples of the metal in the metal-supporting material include metals belonging to the Group 8, 9, and 10 of the periodic table. More preferred are Ni (nickel), Ru (ruthenium), Rh (rhodium), Pd (palladium), and Pt (platinum), and particularly preferred metals are Pd and Pt, especially Pd.

A metal-supporting material in which any of the above-described metals is supported on a support is used as the metal-supporting material, and those in which any of these metals are supported on a support of carbon, alumina, silica-alumina, zeolite, or the like are preferably used. Among these, those in which palladium or platinum is supported on a support are preferable.

Specific examples of the metal-supporting material include Raney nickel, Ru/C, Rh/C, Pd/C, Ir/C, Pt/C, Pd/Al₂O₃, Pd/SiO₂, Pd/TiO₂, Pd/Zeolite, Pt/SiO₂, Pt/Al₂O₃, and Pt/Zeolite.

Next, description will be given of the optically active compound used as an additive, which is a catalyst component, in the present invention. Examples of the optically active compound include amino alcohols, amide alcohols, amino acids, amino esters, amide carboxylic acids, amide esters, and the like. Preferred are optically active amino acids and optically active amino alcohols. Alternatively, salts of these optically active compounds, such as hydrochloric acid salts thereof, can be used.

Specific examples of the optically active amino acids include the following compounds:

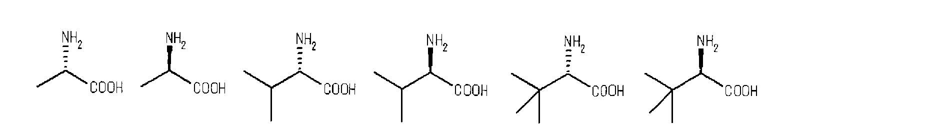

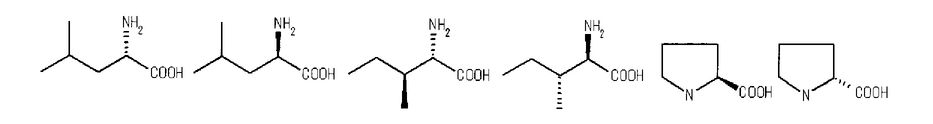

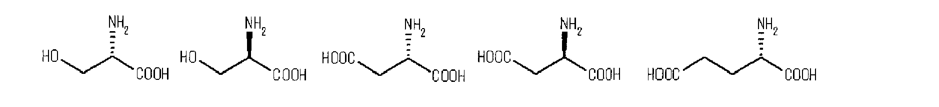

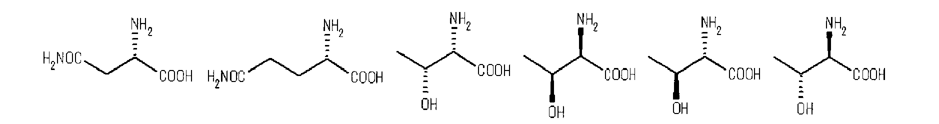

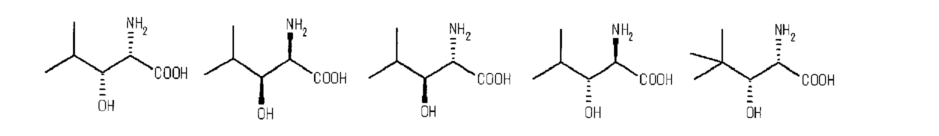

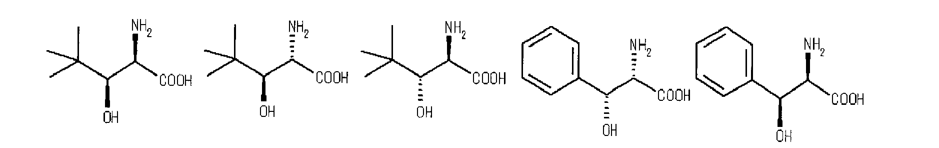

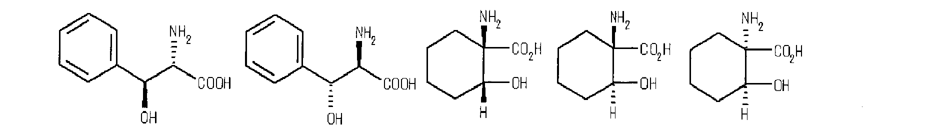

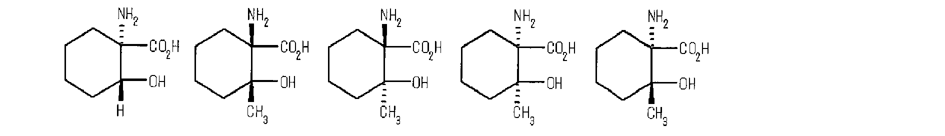

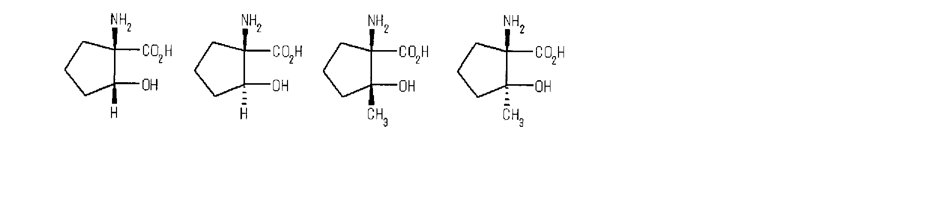

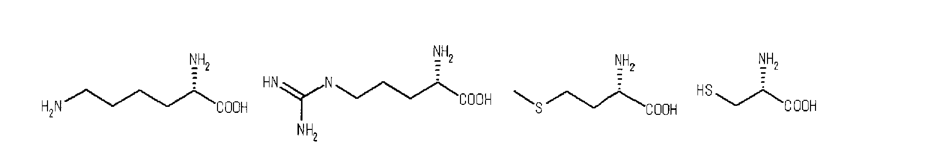

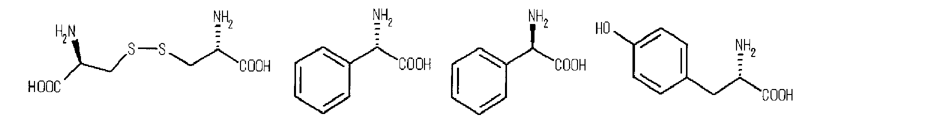

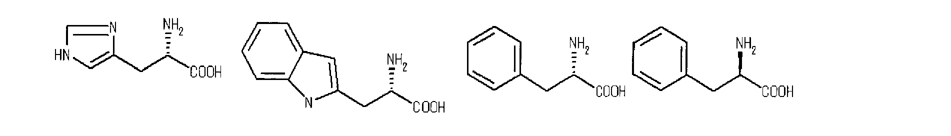

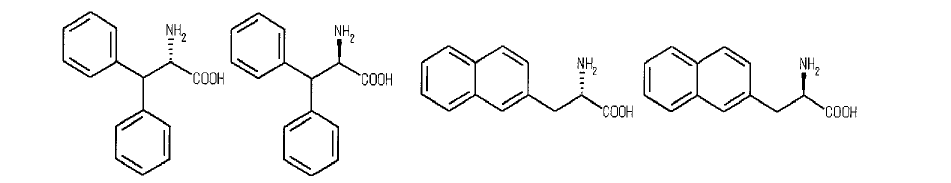

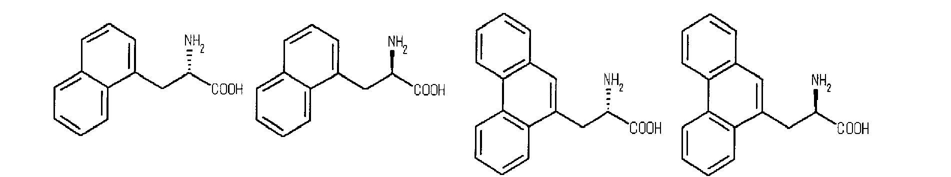

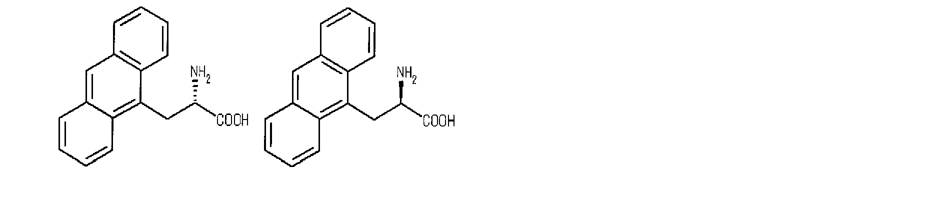

In addition, specific examples of the optically active amino alcohols include the following compounds:

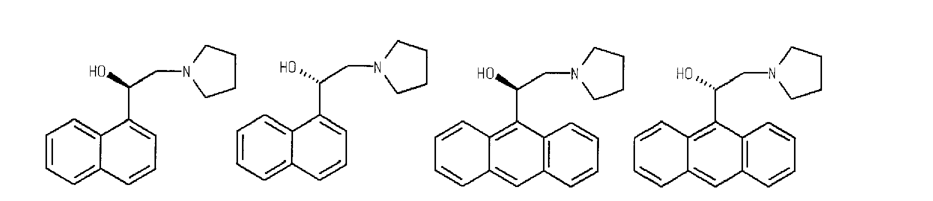

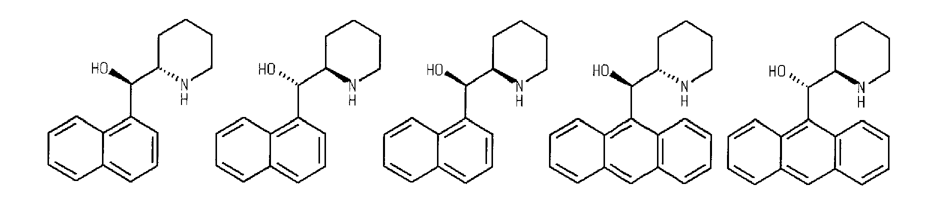

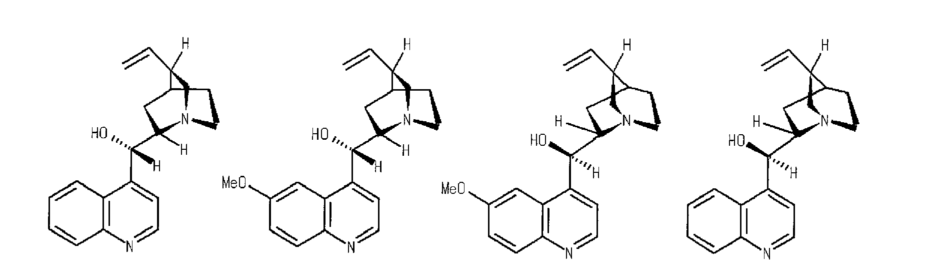

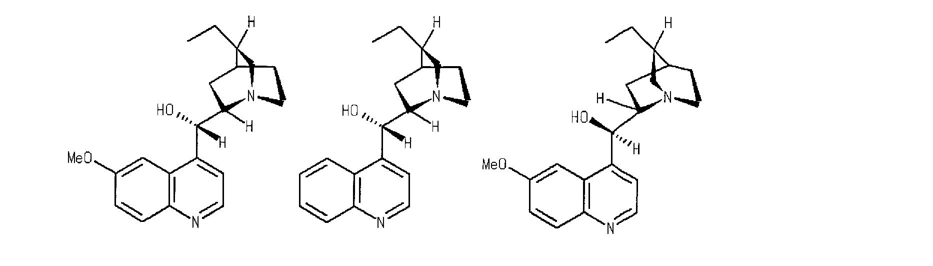

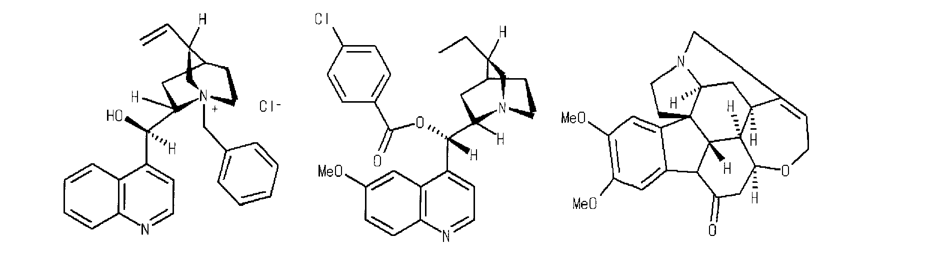

Moreover, in the present invention, an organic base, an organic acid salt, or an inorganic base can be used as an additive, which is another catalyst component, and an inorganic base is preferable.

Specific examples of the organic base include triethylamine, tributylamine, trioctylamine, N,N-dimethylaniline, triphenylamine, and the like.

Specific examples of the organic acid salt include lithium acetate, sodium acetate, potassium acetate, cesium acetate, sodium propionate, potassium propionate, sodium butanoate, potassium butanoate, sodium octanoate, potassium octanoate, ammonium acetate, and the like.

Specific examples of the inorganic base include lithium carbonate, sodium carbonate, potassium carbonate, cesium carbonate, sodium hydrogen carbonate, magnesium carbonate, calcium carbonate, sodium phosphate, potassium phosphate, disodium hydrogen phosphate, dipotassium hydrogen phosphate, sodium dihydrogen phosphate, potassium dihydrogen phosphate, and the like.

In the method of the present invention, an optically active aldehyde can be obtained by asymmetric hydrogenation reaction of the α,β-unsaturated aldehyde in the presence of the above-described catalyst. The amount of the metal-supporting material used as the component of the catalyst of the present invention varies depending on various reaction conditions, and is, for example, 0.01% by weight to 10% by weight, preferably 0.1 % by weight to 5% by weight, relative to the α,β-unsaturated aldehyde, which is the substrate.

Meanwhile, the amount of the optically active compound used as the component of the catalyst of the present invention varies depending on various reaction conditions, and is, for example, 0.01% by weight to 20% by weight, preferably 0.1% by weight to 5% by weight, relative to the α,β-unsaturated aldehyde, which is the substrate.

The method of the present invention can be conducted in the presence or absence of a solvent, and preferably is conducted in the presence of a solvent. Specific preferred examples of the solvent used include aliphatic hydrocarbon-based organic solvents such as hexane, heptane, and octane; alicyclic hydrocarbon-based organic solvents such as cyclohexane and methylcyclohexane; aromatic hydrocarbon-based organic solvents such as benzene, toluene, and xylene; ether-based organic solvents such as diethyl ether, diisopropyl ether, dimethoxyethane, tetrahydrofuran, dioxane, and dioxolane; dimethylformamide; acetonitrile; and the like. It is also possible to use a mixture solvent with any of water; alcohol-based organic solvents such as methanol, ethanol, propanol, isopropanol, and tertiary butanol; halogenated hydrocarbon-based organic solvents such as dichloromethane, dichloroethane, chlorobenzene, and bromotoluene; and the like. Among these solvents, heptane, toluene, and tetrahydrofuran are particularly preferable. The amount of the solvent used can be selected as appropriate in accordance with reaction conditions and the like, and is, for example, 0 to 20 times as much volume, preferably 0 to 5 times as much volume as the α,β-unsaturated aldehyde, which is the substrate.

The method of the present invention is conducted using hydrogen gas as a hydrogen source. The hydrogen pressure is 0.1 MPa to 10 MPa, and preferably 0.1 MPa to 1 MPa. The reaction temperature is 0 to 100°C, and preferably 10 to 40°C. The reaction time varies depending on reaction conditions, and is normally 1 to 30 hours.

The optically active aldehyde obtained as described above can be isolated and purified by ordinarily employed operations such as extraction, recrystallization, and various kinds of chromatography. In addition, regarding the configuration of the optically active aldehyde obtained, a d-isomer or an I-isomer (R-isomer or S-isomer) can be produced by appropriately selecting the configuration of the optically active compound.

### EXAMPLES

Hereinafter, the present invention will be described in detail by using Comparative Examples and Examples. However, the present invention is not limited thereto at all.

For measurement of the products, the gas chromatography method (GLC) was employed. The conditions were as follows:
Analyzer used: G2010 gas chromatograph manufactured by Shimadzu Corporation
Column: DB-WAX (0.25 mm × 30 m) manufactured by Agilent Technologies, Inc. for conversion measurement;
   β-DEX-225 (0.25 mm × 30m) manufactured by Supelco for optical purity
Detector: FID

### (Example 1)

To a 10-ml reaction flask, 1 g (6.57 mmol) of neral, 50 mg (5 wt%) of 5% Pd-C, 5 mg (0.5 wt%) of L-phenylalanine, and 2 ml of toluene were added and stirred. The atmosphere was replaced with a hydrogen atmosphere. After stirring at room temperature for 21 hours, the catalyst was filtered off, and then analysis by gas chromatography was conducted. The conversion of neral to citronellal was 90%. The obtained citronellal was the I-isomer, and had an optical purity of 11.13% e. e.

### (Examples 2 to 28)

Table 1 below shows the results of reactions conducted in the same manner as in Example 1, except that the optically active compounds listed in Table 1 were used instead of L-phenylalanine. Note that, in Example 28, heptane (2 ml) was used as the solvent instead of toluene.

**Table 1**

| Example | Optically active compound | Conversion (%) | Configuration | Optical purity (%ee) |
|---|---|---|---|---|
| 2 | D-Phenylalanine 5 mg (0.5 wt%) | 69.23 | *d* | 12.81 |
| 3 | 3-(2-naphthyl)-L-Alanine 5 mg (0.5 wt%) | 65.31 | / | 10.08 |
| 4 | L-Alanine 5 mg (0.5 wt%) | 75.08 | / | 4.09 |
| 5 | L-Valine 5 mg (0.5 wt%) | 68.23 | / | 7.41 |
| 6 | L-Leucine 5 mg (0.5 wt%) | 81.93 | / | 2.89 |
| 7 | L-tert-Leucine 5 mg (0.5 wt%) | 56.26 | / | 15.82 |
| 8 | L-Isoleucine 5 mg (0.5 wt%) | 74.95 | / | 4.88 |
| 9 | L-Tryptophan 5 mg (0.5 wt%) | 99.56 | / | 2.99 |
| 10 | L-2-Phenylglycine 5 mg (0.5 wt%) | 88.73 | / | 1.64 |
| 11 | L-Histidine 5 mg (0.5 wt%) | 73.93 | | 2.88 |
| 12 | L-Arginine 5 mg (0.5 wt%) | 100 | *d* | 1.16 |
| 13 | L-Cystine 5 mg (0.5 wt%) | 7.98 | *d* | 1.07 |
| 14 | L-Methionine 5 mg (0.5 wt%) | 21.82 | *d* | 1.45 |
| 15 | L-Serine 5 mg (0.5 wt%) | 75.92 | / | 1.90 |
| 16 | D-Threonine 5 mg (0.5 wt%) | 41.2 | *d* | 5.98 |
| 17 | D-Valine 5 mg (0.5 wt%) | 98.9 | *d* | 3.72 |
| 18 | Cinchonidine 5 mg (0.5 wt%) | 100 | / | 5.28 |
| 19 | Cinchonine 5 mg (0.5 wt%) | 100 | *d* | 2.80 |
| 20 | Quinidine 5 mg (0.5 wt%) | 100 | *d* | 1.06 |
| 21 | Quinine 5 mg (0.5 wt%) | 100 | / | 2.95 |
| 22 | Hydrocinchonine 5 mg (0.5 wt%) | 100 | *d* | 3.18 |
| 23 | Hydroquinidine 5 mg (0.5 wt%) | 100 | *d* | 1.00 |
| 24 | Hydroquinine 5 mg (0.5 wt%) | 100 | / | 0.80 |
| 25 | Hydroquinidine 4-chlorobenzoate 5 mg (0.5 wt%) | 100 | / | 2.54 |
| 26 | (8S,9R)-(-)-N-Benzylcinchonidinium chloride 5 mg (0.5 wt%) | 100 | / | 6.79 |
| 27 | Brucine 5 mg (0.5 wt%) | 100 | / | 1.85 |
| 28 | Cinchonidine 5 mg (0.5 wt%) | 100 | / | 2.54 |

### (Examples 29 to 40)

Table 2 below shows the results of reactions conducted in the same manner as in Example 1, except that 1 g (6.57 mmol) of geranial was used as the substrate instead of neral, and the optically active compounds listed in Table 2 were used instead of L-phenylalanine.

**Table 2**

| Example | Optically active compound | Conversion (%) | Configuration | Optical purity (%ee) |
|---|---|---|---|---|
| 29 | L-Phenylalanine 5 mg (0.5 wt%) | 62.02 | *d* | 35.44 |
| 30 | D-Phenylalanine 5 mg (0.5 wt%) | 31.15 | / | 35.05 |
| 31 | D-Diphenylalaine 5 mg (0.5 wt%) | 47.36 | / | 47.81 |
| 32 | 3-(2-naphthyl)-L-Alanine 5 mg (0.5 wt%) | 66.83 | *d* | 35.93 |
| 33 | Cinchonidine 5 mg (0.5 wt%) | 100 | *d* | 3.77 |
| 34 | L-Alanine 5 mg (0.5 wt%) | 49.9 | *d* | 2.35 |
| 35 | L-Valine 5 mg (0.5 wt%) | 40.42 | *d* | 8.86 |
| 36 | L-Leucine 5 mg (0.5 wt%) | 78.48 | *d* | 6.26 |
| 37 | L-tert-Leucine 5 mg (0.5 wt%) | 67.87 | *d* | 33.01 |
| 38 | L-Isoleucine 5 mg (0.5 wt%) | 94.81 | *d* | 7.26 |
| 39 | L-Tryptophan 5 mg (0.5 wt%) | 84.48 | *d* | 14.97 |
| 40 | D-Threonine 5 mg (0.5 wt%) | 47.61 | *d* | 4.56 |
| 41 | O-t-Bu-L-Tyr 5 mg (0.5 wt%) | 25.42 | *d* | 39.83 |
| 42 | p-NO2-L-Phe 5 mg (0.5 wt%) | 86.96 | *d* | 20.91 |
| 43 | o-Me-L-Phe 5 mg (0.5 wt%) | 49.61 | *d* | 50.22 |
| 44 | O-t-Bu-L-Thr 5 mg (0.5 wt%) | 100 | *d* | 21.40 |
| 45 | p-F-L-Phe 5 mg (0.5 wt%) | 73.09 | *d* | 17.31 |
| 46 | L-Cy-Ala 5 mg (0.5 wt%) | 34.53 | *d* | 10.21 |

The optically active compounds of Examples 41 to 46 shown in the table are compounds as follows:

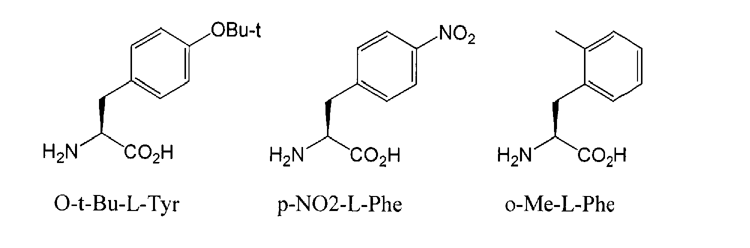

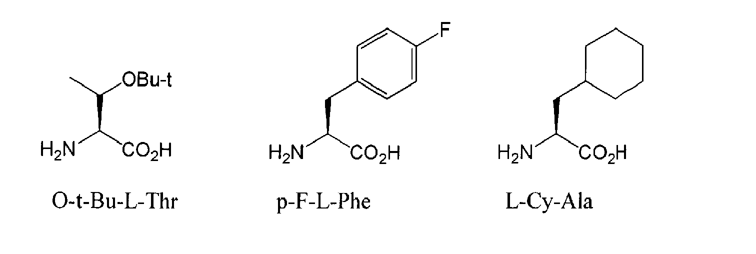

### (Examples 47 to 56)

Table 3 below shows the results of reactions conducted in the same manner as in Example 1, except that 1 g (6.57 mmol) of citral was used as the substrate instead of neral, and the optically active compounds listed in Table 3 were used instead of L-phenylalanine.

**Table 3**

| Example | Optically active compound | Conversion (%) | Configuration | Optical purity (%ee) |
|---|---|---|---|---|
| 47 | L-Phenylalanine 5 mg (0.5 wt%) | 62.13 | *d* | 13.45 |
| 48 | L-tert-Leucine 5 mg (0.5 wt%) | 48.05 | *d* | 12.65 |
| 49 | 3-(2-naphthyl)-L-Alanine 5 mg (0.5 wt%) | 71.67 | *d* | 10.62 |
| 50 | Cinchonidine 5 mg (0.5 wt%) | 100 | / | 0.73 |
| 51 | L-Alanine 5 mg (0.5 wt%) | 56.91 | *d* | 0.46 |
| 52 | L-Valine 5 mg (0.5 wt%) | 57.57 | *d* | 2.47 |
| 53 | L-Leucine 5 mg (0.5 wt%) | 53.13 | *d* | 0.57 |
| 54 | L-Isoleucine 5 mg (0.5 wt%) | 74.5 | *d* | 2.17 |
| 55 | L-Tryptophan 5 mg (0.5 wt%) | 80.62 | *d* | 1.85 |
| 56 | D-Threonine 5 mg (0.5 wt%) | 51.99 | *d* | 6.49 |

### (Examples 57 to 70)

Table 4 below shows the results of reactions conducted in the same manner as in Example 1, except that 1 g (6.57 mmol) of geranial was used as the substrate instead of neral, and 0.5% by weight of an inorganic base or an organic base was added as an additive to geranial.

**Table 4**

| Example | Additive | Conversion (%) | Configuration | Optical purity (%ee) |
|---|---|---|---|---|
| 57 | Li₂CO₃ 5 mg (0.5 wt%) | 93.16 | *d* | 12.41 |
| 58 | Na₂CO₃ 5 mg (0.5 wt%) | 89.46 | *d* | 39.31 |
| 59 | K₂CO₃ 5 mg (0.5 wt%) | 78.27 | *d* | 33.21 |
| 60 | CS₂CO₃ 5 mg (0.5 wt%) | 53.19 | *d* | 24.56 |
| 61 | NaHCO₃ 5 mg (0.5 wt%) | 73.82 | *d* | 31.02 |
| 62 | MgCO₃ 5 mg (0.5 wt%) | 17.56 | *d* | 29.30 |
| 63 | CaCO₃ 5 mg (0.5 wt%) | 46.58 | *d* | 29.28 |
| 64 | Na₃PO₄ 5 mg (0.5 wt%) | 68.09 | *d* | 32.87 |
| 65 | Na₂HPO₄ 5 mg (0.5 wt%) | 31.34 | *d* | 39.76 |
| 66 | NaH₂PO₄ 5 mg (0.5 wt%) | 52.87 | *d* | 4.88 |
| 67 | Et₃N 5 mg (0.5 wt%) | 21.17 | *d* | 14.95 |
| 68 | AcONa 5 mg (0.5 wt%) | 54.73 | *d* | 29.24 |
| 69 | NaBF₄ 5 mg (0.5 wt%) | 17.28 | *d* | 28.21 |
| 70 | AcONH₄ 5 mg (0.5 wt%) | 15.41 | *d* | 4.17 |

### (Examples 71 to 73)

Table 5 below shows the results of reactions conducted in the same manner as in Example 58, except that 1 g (6.57 mmol) of geranial was used as the substrate instead of neral, and the optically active compounds listed in Table 5 were used instead of L-phenylalanine.

**Table 5**

| Example | Optically active compound | Conversion (%) | Configuration | Optical purity (%ee) |
|---|---|---|---|---|
| 71 | L-tert-Leucine 5 mg (0.5 wt%) | 79.76 | *d* | 28.52 |
| 72 | 3-(2-naphthyl)-L-Alanine 5 mg (0.5 wt%) | 80.98 | *d* | 31.52 |
| 73 | 3-(1-naphthyl)-L-Alanine·HCI 5 mg (0.5 wt%) | 44.79 | *d* | 44.20 |

### (Examples 73 to 77)

Table 6 below shows the results of reactions conducted in the same manner as in Example 1, except that 1 g (6.57 mmol) of geranial was used as the substrate instead of neral, and the metal-supporting materials listed in Table 6 were used instead of 5% Pd-C.

**Table 6**

| Example | Metal-supporting material | Conversion (%) | Configuration | Optical purity (%ee) |
|---|---|---|---|---|
| 74 | 5% Pd-SiO₂ 25 mg (2.5 wt%) | 79.32 | *d* | 15.25 |
| 75 | 5% Pd-Al₂O₃ 25 mg (2.5 wt%) | 61.01 | *d* | 9.97 |
| 76 | Lindlar 25 mg (2.5 wt%) | 16.62 | *d* | 2.92 |
| 77 | 5% Pt-Al₂O₃ 25 mg (2.5 wt%) | 99.94 | *d* | 5.06 |
| 78 | 5% Rh-C 25 mg (2.5 wt%) | 44.59 | *d* | 2.63 |

### (Examples 79 to 84)

Table 7 below shows the results of reactions conducted in the same manner as in Example 29, except that the reaction solvents listed in Table 7 were used instead of toluene.

**Table 7**

| Example | Reaction solvent | Conversion (%) | Configuration | Optical purity (%ee) |
|---|---|---|---|---|
| 79 | Tetrahydrofuran (2 ml) | 24.54 | *d* | 32.82 |
| 80 | Isopropanol (2 ml) | 44.62 | *d* | 25.80 |
| 81 | Methanol (2 ml) | 48.51 | *d* | 22.52 |
| 82 | Heptane (2 ml) | 40.65 | *d* | 38.53 |
| 83 | Ethyl acetate (2 ml) | 37.04 | *d* | 19.86 |
| 84 | Acetonitrile (2 ml) | 100 | *d* | 2.05 |

### (Example 85)

To a 10-ml reaction flask, 1 g (6.57 mmol) of α-methyl-trans-cinnamaldehyde, 50 mg (5 wt%) of 5% Pd-C, 5 mg (0.5 wt%) of L-phenylalanine, and 2 ml of toluene were added and stirred. The atmosphere was replaced with a hydrogen atmosphere. After stirring at room temperature for 21 hours, the catalyst was filtered off, and then analysis by gas chromatography was conducted. The conversion of the substrate was 23%, and the optical purity of obtained 2-methyl-3-phenylpropionaldehyde was 10.20% e.e.

## Claims

1. A solid catalyst for asymmetric hydrogenation of an α,β-unsaturated aldehyde, comprising:
a metal-supporting material in which at least one metal selected from the group consisting of elements of Groups 8 to 10 of a periodic table is supported on a support; and
an optically active compound.

2. The solid catalyst according to claim 1, further comprising an organic base, an organic acid salt, or an inorganic base.

3. The solid catalyst according to claim 1 or 2, wherein the optically active compound is an amino acid, an amino alcohol, a salt of an amino acid and an acid, or a salt of an amino alcohol and an acid.

4. The solid catalyst according to any one of claims 1 to 3, wherein the metal is selected from the group consisting of nickel, ruthenium, rhodium, palladium, and platinum.

5. A method for producing an optically active aldehyde, comprising the step of:
performing asymmetric hydrogenation of an α,β-unsaturated aldehyde by using the solid catalyst according to any one of claims 1 to 4, the α,β-unsaturated aldehyde being represented by the following general formula (1): 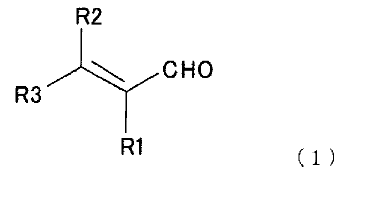 wherein R¹, R² and R³ each independently represent a hydrogen atom, an alkyl group which may have a substituent, a cycloalkyl group which may have a substituent, an alkenyl group which may have a substituent, or an aryl group which may have a substituent; R¹ and R³, or R² and R³ may form a ring, provided that R² and R³ are mutually different groups, and that two groups of R¹, R², and R³ are not simultaneously hydrogen atoms,
the optically active aldehyde being represented by the following general formula (2): 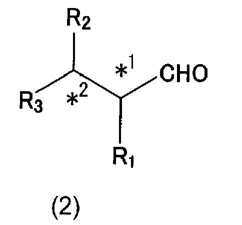 wherein R¹, R² and R³ have the same meanings as those in the formula (1); and *¹ and *² represent asymmetric carbon atoms; provided that when R¹ is hydrogen, *¹ is not an asymmetric carbon atom, and that when R² or R³ is hydrogen, *² is not an asymmetric carbon atom.

6. The production method according to claim 5, wherein the α,β-unsaturated aldehyde is geranial, neral, or citral (a mixture of geranial and neral).
